# EUROPEAN PATENT APPLICATION

(11) **EP 1 413 261 A1**
(43) Date of publication of application: **28.04.2004**
(21) Application number: 03023999.0
(22) Date of filing: 22.10.2003
(51) Int. Cl.: A61F 2/06

(54) **Stent with detachable ends**

(30) Priority: 23.10.2002 US 420695 P
(71) Applicant: Medtronic Vascular, Inc., Santa Rosa, California 95403 (US)
(72) Inventor: Sundar, Ron, Santa Rosa, CA 95404 (US)
(74) Representative: Hughes, Andrea Michelle

(57) **Abstract**

The stent with detachable ends of the present invention avoids problems from pooling or bridging between a stent and a fixture supporting the stent when a coating is applied. A preliminary stent 150 can be held by at least one retainer 152 to allow coating to be applied to the preliminary stent. The preliminary stent comprises a detachable portion (154) made of detachable segments and a permanent portion (158) made of permanent segments. After the coating (125) has been applied, the detachable portion can be removed from the permanent portion to form the final stent. Removing the detachable portion removes any detachable segments where the liquid coating may have pooled at contact points between the retainer and the detachable segments.

## Description

### TECHNICAL FIELD

The technical field of this disclosure is medical implant devices, particularly, a stent having detachable ends.

### BACKGROUND OF THE INVENTION

Stents are generally cylindrical shaped devices that are radially expandable to hold open a segment of a blood vessel or other anatomical lumen after implantation into the body lumen. Stents have been developed with coatings to deliver drugs or other therapeutic agents.

Stents are used in conjunction with balloon catheters in a variety of medical therapeutic applications including intravascular angioplasty. For example, a balloon catheter device is inflated during PTCA (percutaneous transluminal coronary angioplasty) to dilate a stenotic blood vessel. The stenosis may be the result of a lesion such as a plaque or thrombus. After inflation, the pressurized balloon exerts a compressive force on the lesion thereby increasing the inner diameter of the affected vessel. The increased interior vessel diameter facilitates improved blood flow. Soon after the procedure, however, a significant proportion of treated vessels re-narrow.

To prevent restenosis, short flexible cylinders, or stents, constructed of metal or various polymers are implanted within the vessel to maintain lumen size. The stent acts as a scaffold to support the lumen in an open position. Various configurations of stents include a cylindrical tube defined by a mesh, interconnected stents or like segments. Some exemplary stents are disclosed in U.S. Patent No. 5,292,331 to Boneau, U.S. Patent No. 6,090,127 to Globerman,

U.S. Patent No. 5,133,732 to Wiktor, U.S. Patent No. 4,739,762 to Palmaz and U.S. Patent No. 5,421,955 to Lau. Balloon-expandable stents are mounted on a collapsed balloon at a diameter smaller than when the stents are deployed.

Stents have been used with coatings to deliver drug or other therapy at the site of the stent. The coating can be applied as a liquid containing the drug or other therapeutic agent dispersed in a polymer/solvent matrix. The liquid coating then dries to a solid coating upon the stent. The coating can be applied by dipping or spraying the stent while spinning or shaking the stent to achieve a uniform coating. Combinations of the various application techniques can also be used. When the coating is applied, the stent is generally supported on a fixture, such as a mandrel or similar device that allows the stent to be immersed in or sprayed with the coating material. The stent is retained on the fixture to prevent the stent from sliding. Cones aligned with the long axis and inserted in the ends of the stent, supporting pins, or clips can be used to retain the stent. Possible retainer materials that can be used to reduce liquid adhesion to the retainer include Delrin™, Teflon™, or stainless steel.

Despite the slick surface of the retainer, the coating still pools and bridges between the retainer and the stent, i.e., excess liquid collects at the contact points between the retainer and the stent. The complex geometry of the stent provides many contact points between the retainer and the stent, and thus, many points where pooling is a problem. As the concentration of the coating solution increases due to higher resin solids and/or increased drug loading, the pooling becomes more pronounced.

Pooling or bridging creates a number of manufacturing problems. It is desirable'to have a uniform coating to assure a uniform therapeutic dose to the patient. The pooling point can produce a thick spot in the coating if it dries on the stent and a thin spot in the coating if it pulls away with the retainer. The pooling also complicates the drying process because the thicker liquid at the pooling point dries more slowly than the thinner, uniform coating on the rest of the stent .

It would be desirable to have a stent having detachable ends that would overcome the above disadvantages.

### SUMMARY OF THE INVENTION

One aspect of the present invention provides a stent as defined in claim 1.

In another aspect, the invention provides a stent delivery system as defined in claim 4.

The invention also provides a method and system for making such a stent.

An object of the invention is to avoid pooling of coating applied to the stent.

Another object of the present invention is to provide a stent with a uniform coating thickness.

Another object of the present invention is to provide a stent affording precise control over drug delivery.

Another object of the present invention is to provide a stent which uses normal drying procedures.

The foregoing and other features and advantages of the invention will become further apparent from the following detailed description of the presently preferred embodiments, given by way of example only, read in conjunction with the accompanying drawings. The detailed description and drawings are merely illustrative of the invention, rather than limiting the scope of the invention being defined by the appended claims.

### BRIEF DESCRIPTION OF THE DRAWINGS

FIG. 1 shows a stent delivery system made in accordance with the present invention.
FIG. 2 shows a preliminary stent with retainers made in accordance with the present invention.
FIG. 3 shows a stent made in accordance with the present invention.

### DETAILED DESCRIPTION OF THE PRESENTLY PREFERRED EMBODIMENT

The stent with detachable ends of the present invention avoids problems from pooling or bridging between a stent and a fixture supporting the stent when a coating is applied. Coating may be applied in any number of ways, including spraying, dipping to mention only a few. The exact way used depends on the stent design, the polymer matrix as well as the drug and respective solvents and dosage criteria. A preliminary stent can be held by at least one retainer to allow coating to be applied to the preliminary stent. The preliminary stent comprises a detachable portion made of detachable segments and a permanent portion made of permanent segments. After the coating has been applied, the detachable portion can be removed from the permanent portion to form the final stent. Removing the detachable portion removes any detachable segments where the coating may have pooled at contact points between the retainer and the detachable segments.

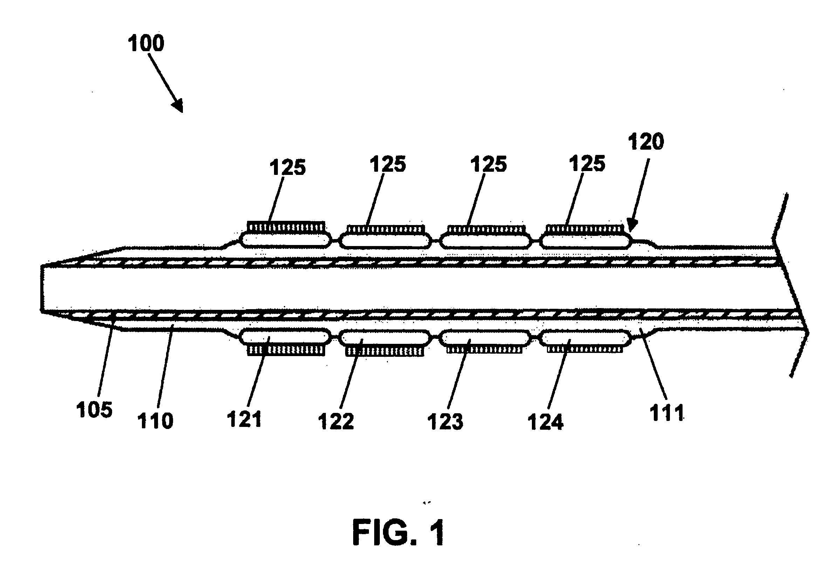

FIG. 1 shows a stent delivery system made in accordance with the present invention. The stent delivery system 100 includes a catheter 105, a balloon 110 operably attached to the catheter 105, and a stent 120 disposed on the balloon 110. The balloon 110, shown in a collapsed state, may be any variety of balloons capable of expanding the stent 120. The balloon 110 may be manufactured from any sufficiently elastic material such as polyethylene, polyethylene terephthalate (PET), nylon, or the like. In one embodiment, the balloon 110 may include retention means 111, such as mechanical or adhesive structures, for retaining the stent 120 until it is deployed. The catheter 105 may be any variety of balloon catheters, such as a PTCA (percutaneous transluminal coronary angioplasty) balloon catheter, capable of supporting a balloon during angioplasty. The stent 120 may be any variety of implantable prosthetic devices capable of carrying a coating known in the art. In one embodiment, the stent 120 may have a plurality of identical cylindrical stent segments placed end to end. Four stent segments 121, 122, 123, and 124 are shown, and it will be recognized by those skilled in the art that an alternative number of stent segments may be used. The stent 120 includes at least one coating layer 125 carrying a therapeutic agent, which can be applied to the stent 120 by dipping or spraying the stent 120 with a coating liquid, or applying the coating liquid with a combination of methods. The coating can be applied as a liquid containing the drug or other therapeutic agent dispersed in a polymer/solvent matrix. The coating layer 125 is merely exemplary, and it should be recognized that other coating configurations, such as multiple coating layers, are possible. In another embodiment, the therapeutic agent can be omitted from the coating and the coating included for its mechanical properties.

The coating layer 125 may be a polymer including, but not limited to, urethane, polycaprolactone (PCL), polymethylmethacrylate (PMMA), combinations of the above, and the like. Suitable solvents that may be used to form the liquid coating include, but are not limited to, acetone, ethyl acetate, tetrahydrofuran (THF), chloroform, N?methylpyrrolidone (NMP), combinations of the above, and the like. Suitable therapeutic agents include, but are not limited to, antiangiogenesis agents, antiendothelin agents, antimitogenic factors, antioxidants, antiplatelet agents, antiproliferative agents, antisense oligonucleotides, antithrombogenic agents, calcium channel blockers, clot dissolving enzymes, growth factors, growth factor inhibitors, nitrates, nitric oxide releasing agents, vasodilators, virus-mediated gene transfer agents, agents having a desirable therapeutic application, combinations of the above, and the like. Specific examples of therapeutic agents include abciximab, angiopeptin, colchicine, eptifibatide, heparin, hirudin, lovastatin, methotrexate, streptokinase, taxol, ticlopidine, tissue plasminogen activator, trapidil, urokinase, and growth factors VEGF, TGF-beta, IGF, PDGF, and FGF.

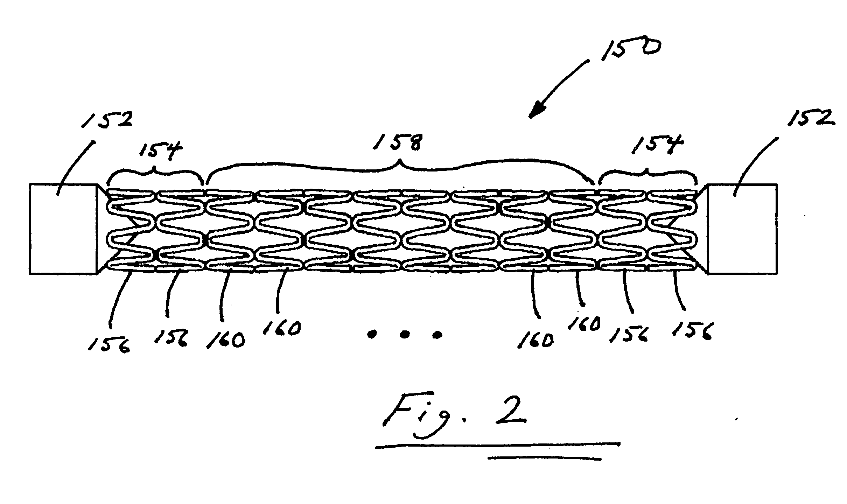

FIG. 2 shows a preliminary stent with retainers made in accordance with the present invention. The preliminary stent 150 can be held by at least one retainer 152 to allow liquid coating to be applied to the preliminary stent 150. The preliminary stent 150 comprises a detachable portion 154 made of detachable segments 156 and a permanent portion 158 made of permanent segments 160. After the liquid coating has been applied, the detachable portion 154 can be removed from the permanent portion 158 to form the final stent. Removing the detachable portion 154 removes any detachable segments 156 where the liquid coating may have pooled at contact points between the retainer 152 and the detachable segments 156.

The preliminary stent 150 is conventional to stents generally and may be made of a wide variety of medical implantable materials, such as stainless steel, nitinol, tantalum, ceramic, nickel, titanium, aluminum, polymeric materials, tantalum, MP35N, stainless steel, titanium ASTM F63-83 Grade 1, niobium, high carat gold K 19-22, or combinations of the above. The preliminary stent 150 may be formed through various methods as well. The preliminary stent 150 may be welded, molded, cut from a tube, or consist of filaments or fibers which are wound or braided together in order to form a continuous structure. Depending on the material, the stent may be self-expanding, or be expanded by a balloon or some other device.

The pattern of the permanent segments 160 may be W-shaped or may be a more complex shape with the elements of one segment continuing into the adjacent segment. The detachable portion 154 may continue the pattern of the permanent segments 160, or may be a simpler pattern, since the detachable portion 154 is discarded after removal and only the permanent portion 158 used.

The retainer 152 may be any device capable of holding the preliminary stent 150 in place as the coating is applied. If the preliminary stent 150 is to be subject to vigorous action as the coating is applied, such as dipping or spinning, a pair of retainers 152 with one retainer in each end of the preliminary stent 150 may be used to secure the preliminary stent 150. If the coating is applied as a low pressure spray or by vapor deposition, a single retainer in one end of the preliminary stent 150 may be sufficient.

Although a pair of conical retainers 152 is illustrated, those skilled in the art will appreciated that many possible means for retaining the preliminary stent 150 are possible: a spring-loaded clip can fit around the end and grasp the inside and outside of the detachable portion 154; a truncated cone can be inserted like a cork in the detachable portion 154; a cylindrical support pin can be inserted in the detachable portion 154 along the long axis of the preliminary stent 150; or the cylindrical support pin can be used with a cross pin passing transversely through the preliminary stent 150 and the support pin to further fix the preliminary stent 150. The retainer may be inside or outside the detachable portion 154, or both inside and outside. Any method of retaining the preliminary stent 150 that limits contact of retainer 152 to the detachable portion 154 of the preliminary stent 150 may be used.

To prepare the preliminary stent 150, at least one retainer 152 is inserted in the detachable portion 154 of the preliminary stent 150. The coating is applied and allowed to dry to the desired hardness. The preliminary stent 150 may then be removed from the retainer 152, or in an alternate embodiment, the retainer 152 may be used to maintain a hold on the preliminary stent 150 for the removal of the detachable portion 154. The detachable portion 154 of the preliminary stent 150 may then be removed from the permanent portion 158, by laser cutting, mechanical cutting, or the like. The detachable portion 154 may be discarded and the permanent portion 158 retained as the final stent. The cutting may be performed where two stent segments join, or may be through one of the segments, depending on the stent geometry. The cut edges may be smoothed and polished to remove any sharp edges that could cause damage during implantation in a body lumen.

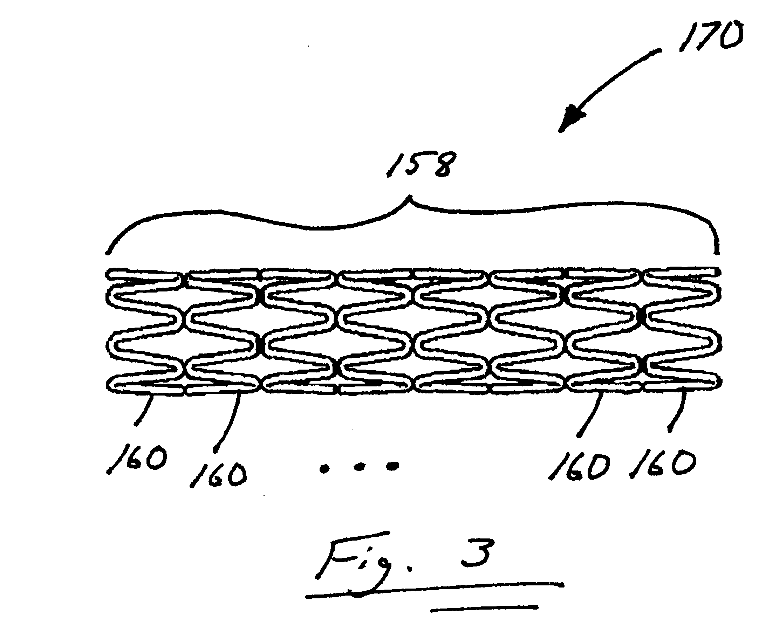

FIG. 3, in which like elements share like reference numbers with FIG. 2, shows a stent made in accordance with the present invention. The final stent 170 comprises the permanent portion 158 made of permanent segments 160. The detachable portion made of detachable segments as shown in FIG. 2 has been removed. The final stent 170 may be installed in the stent delivery system of FIG. 1 for implantation in a body lumen.

It is important to note that FIGS. 1 - 3 illustrate specific, preferred applications and embodiments of the present invention by way of example only.

## Claims

1. A coated stent comprising:
a stent (120);
a coating (125) disposed on the stent, the coating applied to a preliminary stent (150) comprising a permanent portion (158) and a detachable portion (154) while retaining the preliminary stent by the detachable portion, the stent formed from the permanent portion by removing the detachable portion.

2. The coated stent of claim 1 wherein the coating includes a therapeutic agent.

3. The coated stent of claim 1 wherein the coating is a polymer.

4. A stent delivery system comprising:
a catheter (105);
a balloon (110) operably attached to the catheter; and
a coated stent (120) as claimed in any of claims 1 to 3, disposed on the balloon.

5. A method for producing a stent comprising:
providing a preliminary stent (150) comprising a permanent portion (158) and a detachable portion (154); retaining the preliminary stent by the detachable portion;
applying a coating (125) to the preliminary stent; and
detaching the detachable portion from the permanent portion.

6. The method of claim 5 further comprising finishing the end of the permanent portion which was detached from the permanent portion.

7. The method of claim 5 or 6 further comprising drying the coating (125).

8. The method of claim 5, 6 or 7 wherein detaching the detachable portion (154) from the permanent portion (158) further comprises detaching the detachable portion from the permanent portion by laser cutting.

9. The method of claim 5, 6 or 7 wherein detaching the detachable portion (154) from the permanent portion (158) comprises detaching the detachable portion from the permanent portion by mechanical cutting.

10. The method of any of claims 5 to 9 wherein applying a coating to the preliminary stent comprises spraying a coating on the preliminary stent.

11. The method of any of claims 5 to 9 wherein applying a coating to the preliminary stent comprises dipping the preliminary stent to form a coating.

12. The method of any of claims 5 to 11 wherein the detachable portion (154) further comprises a first detachable portion and a second detachable portion; and retaining the preliminary stent by the detachable portion further comprises retaining the preliminary stent by the first detachable portion and the second detachable portion.

13. The method of any of claims 5 to 12 wherein the coating includes a therapeutic agent.

14. The method of any of claims 5 to 12 wherein the coating is a polymer.

15. A system for producing a stent comprising:
a preliminary stent (150) comprising a permanent portion (158) and a detachable portion (154);
means (152) for retaining the preliminary stent by the detachable portion;
means for applying a coating (125) to the preliminary stent; and
means for detaching the detachable portion from the permanent portion.

16. The system of claim 15 further comprising means for finishing the end of the permanent portion which was detached from the permanent portion.

17. The system of claim 15 or 16 further comprising means for drying the coating.

18. The system of claim 15, 16 or 17 wherein the detachable portion further comprises a first detachable portion and a second detachable portion; and means for retaining the preliminary stent by the detachable portion further comprises means for retaining the preliminary stent by the first detachable portion and the second detachable portion.
